# EUROPÄISCHE PATENTSCHRIFT

(11) **EP 0 104 491 B1**
(45) Veröffentlichungstag und Bekanntmachung des Hinweises auf die Patenterteilung: **17.12.1986**
(21) Anmeldenummer: 83108622.8
(22) Anmeldetag: 01.09.1983
(51) Int. Cl.: C07C 69/54, C07C 69/60, C07C 69/753, C07C 69/80, C07C 125/073, A61K 6/08

(54) **Neue Diacryl- und Dimethacrylester und ihre Verwendung**
Acrylic and methacrylic acid diesters and their use
Diesters des acides acrylique et méthacrylique et leur utilisation

(30) Priorität: 24.09.1982 US 422727
(43) Veröffentlichungstag der Anmeldung: 04.04.1984
(73) Patentinhaber: Blendax-Werke R. Schneider GmbH & Co., D-6500 Mainz (DE)
(72) Erfinder: Orlowski, Jan A., Dr., Altadena California, 91001 (US); Butler, David V., West Covina California, 91791 (US)

## Beschreibung

Die Erfindung bezieht sich auf neue, polymerisierbare Di(meth)acryl Verbindungen und ihre Anwendung in Bindemitteln, besonders bei Dentalmaterialien, sowie auf dentale Restorations- und Füllungsmaterialien, die diese neuen Verbindungen enthalten.

Es gibt zahlreiche polymerisierbare Verbindungen, die mehr als eine Doppelbindung im Molekül haben. Diese Verbindungen werden zu verschiedenen Zwecken benutzt, besonders als Bindemittel für die Herstellung verschiedener Klebstoffe, u.a. in der Medizin und Zahnmedizin, zur Herstellung dentaler Zemente, dentaler Restorations- und Füllungsmaterialien, dentaler Versiegelungsmaterialien, orthopädischer und orthodontischer Klebstoffe, etc.

Es wurde nun gefunden, daß eine neue Klasse von Monomeren, die durch Reaktion von aliphatischen, aromatischen oder cycloaliphatischen Dicarbonsäureanhydriden mit Diolen und Glycidyl(meth)acrylat erhalten wird, und deren Carbamate besonders geeignet sind als Bindemittel und Klebstoffe, besonders in den oben genannten Anwendungsgebieten.

Gegenstand der Erfindung sind also neue Verbindungen der allgemeinen Formel 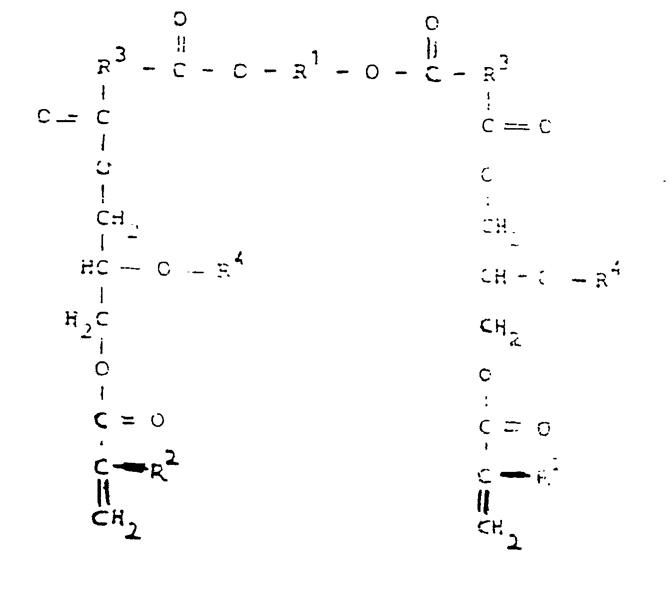 wobei R¹ eine Ethylen-, Propylen-, Butylen- oder Hexamethylengruppe, eine Phenylen-, Toluylen-, Methylenbisphenyl-, Propylenbisphenyl-, Cyclohexan-, Dicyclopentandimethylen-, Methylenbiscyclohexyl-oder eine Propylenbiscyclohexylgruppe, R² H oder eine Methylgruppe, R³ einen -CH=CH-, -CH₂CH₂-, -CH₂CH₂CH₂-, -CH₂CH₂CH₂CH₂- Rest, einen gegebenenfalls substituierten oder hydrierten Benzolring oder einen Cyclohexan-, Cyclohexen- oder Cisnorbornenrest und R⁴ H oder 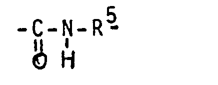 bedeuten, wobei R⁵ einen n-Butyl-, Isobutyl-, Hexyl-, gegebenenfalls substituierten Phenyl- oder Cyclohexylrest darstellt.

R² ist ein Wasserstoffatom oder eine Methylgruppe; die Methacrylverbindung wird bevorzugt.

R³ steht bevorzugt für eine -CH=CH-Gruppe (abgeleitet von Maleinsäure), eine Benzolgruppe (abgeleitet von Phthalsäure), eine Tetrohydrobenzolgruppe (abgeleitet von 4-Cyclohexen-1,2-dicarbonsäure), eine Cyclohexangruppe (abgeleitet von Cyclohexan-1,2-dicarbonsäure) oder einer Cis-Norbonen-Gruppe (abgeleitet von Cis-Norbornendicarbonsäure), jedoch sind beispielsweise auch Malonsäure-, Bernsteinsäure-, Glutarsäure-und Adipinsäureverbindungen geeignet.

R⁴ ist Wasserstoff oder ein Carbaminsäurerest, 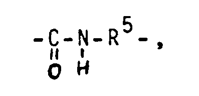 wobei
R⁵ ein n- oder Iso-Butyl-, Hexyl- oder gegebenenfalls substituierter Phenyl- oder Cyclohexylrest sein kann.

Die Herstellung der neuen polymerisierbaren Verbindungen gemäß der Erfindung kann durch folgendes Verfahren erfolgen:
Ein Anhydrid einer aliphatischen, cycloaliphatischen oder aromatischen Dicarbonsäure wird verestert mit einem aliphatischen, aromatischen oder cycloaliphatischen Diol, vorzugsweise bei Temperaturen zwischen ca. 120° C - ca. 170° C.

Dann läßt man diesen Ester mit Glycidylacrylat oder Glycidylmethacrylat zu einem entsprechenden 3-(Meth)Acroyl-2-hydroxy-propylester reagieren.

Dieses Produkt kann in Gegenwart eines bekannten Katalysators bei ca. 40°C - ca. 100°C mit einem Isocyanat zum entsprechenden Carbamat umgesetzt werden.

Die GB-A Nr. 2,079,297 offenbart ein härtbares, dentales Füllungsmaterial, das als Harzbestandteile Reaktionsprodukte aus Diisocyanaten und Hydroxyalkyldiacrylaten und -dimethacrylaten der allgemeinen Formel 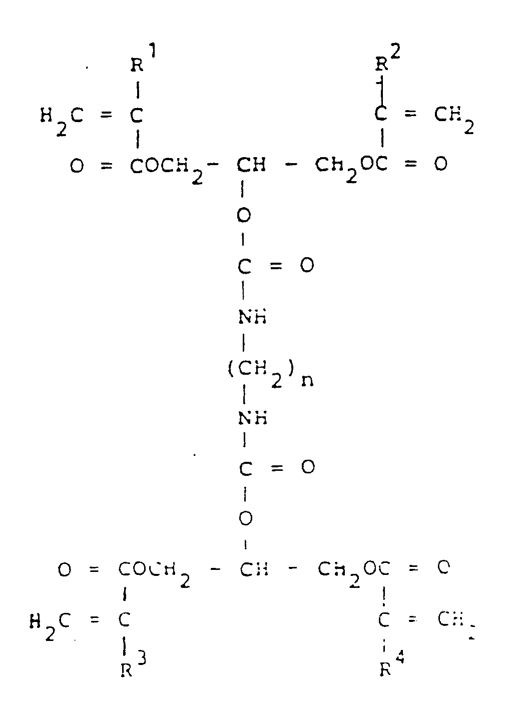 enthält,
wobei R¹, R², R³ und R⁴ Wasserstoff oder eine Methylgruppe bedeuten und n eine Zahl von 2 bis 10 ist.

Jedoch erfüllen die Eigenschaften der gehärteten dentalen Füllungsmaterialien, die solche Monomere enthalten, nicht die Anforderungen der zahnärztlichen Praxis, besonders was die mechanischen Eigenschaften und insbesondere die Härte betrifft.

Die Produkte gemäß der Erfindung sind prinzipiell geeignet für all jene Anwendungen, wo Monomere mit mehr als einer polymerisierbaren Doppelbindung in einem Molekül verwendet werden. Wie eingangs ausgeführt, sind sie besonders geeignet für den Gebrauch in der Medizin und der Zahnmedizin.

In den letzten Jahren haben in der konservierenden Zahnmedizin die sogenannten "Composites" eine wachsende Bedeutung erlangt, da diese Produkte einfach und sicher vom Zahnarzt anzuwenden sind und von den Patienten gut vertragen werden. Sie nähern sich dem Ziel, die Amalgamfüllungsmaterialien zu verdrängen, die aus physiologischen Gründen kritisiert werden.

Diese Composites bestehen gewöhnlich aus anorganischen Füllstoffen und polymerisierbaren Verbindungen.

Nachteile der Composites im Vergleich zu konventionellen Füllungsmaterialien sind ihre Abrieb- und Schrumpfungsanfälligkeit und ihre Wasserabsorption. Sekundärkaries kann, verursacht durch die mögliche Schrumpfung zwischen dem Rand der Kavität und der Füllung im Zahn, die Folge sein.

Deshalb wurden zahlreiche Versuche unternommen, Composites herzustellen, die keinen oder nur einen geringen Schrumpfungsgrad besitzen, eine niedrige Wassersorption und gute mechanische Eigenschaften, besonders hinsichtlich der Härte, aufweisen und farbstabil sind.

Aus der DE-B 2 714 538 sind bereits Methacrylsäureester von tricyclischen Decanolen bekannt, die zur Herstellung von Klebstoffen und Dichtmitteln Verwendung finden sollen. Diese Mittel sollen unter Sauerstoffausschluß erhärten.

Für den Fachmann ist damit der Einsatz solcher Monomerer, die sich im übrigen strukturell von den erfindungsgemäßen Monomeren unterscheiden, in dentalen Füllungs- und Restorationsmaterialien weitgehend ausgeschlossen, da diese gerade in Anwesenheit von Luftsauerstoff bei geöffnetem Mund des Patienten in der gefüllten Kavität ausgehärtet werden müssen.

Aus der US-A 4 337 349, die auf die Erfinder zurückgeht, ist die Verwendung der Tricylodecanmethacrylsäureester nach der DE-B 2 714 538 auch in dentalen Restorationsmaterialien bekannt. Die diese von den erfindungsgemäßen Monomeren strukturell abweichenden Monomeren enthaltenden Füllungsmaterialien sollen in ausgehärtetem Zustand vorteilhafte mechanische Eigenschaften aufweisen. Spätere Untersuchungen haben jedoch gezeigt, daß die mit diesen Monomeren erhaltenen Füllungen optisch nicht so ansprechend sind wie ursprünglich vermutet, d.h., mit zunehmendem Alter der Füllungen treten Verfärbungen auf. Es bestand daher ein Bedürfnis, weitere geeignete Monomere aufzufinden, die zumindest gleich gute mechanische Eigenschaften bei der Anwendung in dentalen Füllungsmaterialien, jedoch auch bei der Alterung keine Verfärbung der Füllung im Mund ergeben.

Überraschenderweise läßt sich diese Aufgabe durch Verwendung der erfindungsgemäß definierten (Meth)Acrylsäureester lösen.

Diese Eigenschaften können durch einen hohen Anteil von anorganischen Füllstoffen in den Zusammensetzungen erreicht werden. Jedoch ist der maximal mögliche füllstoffanteil weitgehend von den Eigenschaften der Monomeren, die in der Zusammensetzung vorliegen, abhängig.

Konventionelle, auf dem Markt befindliche Composites haben im allgemeinen nach der Aushärtung die folgenden mechanischen Eigenschaften:

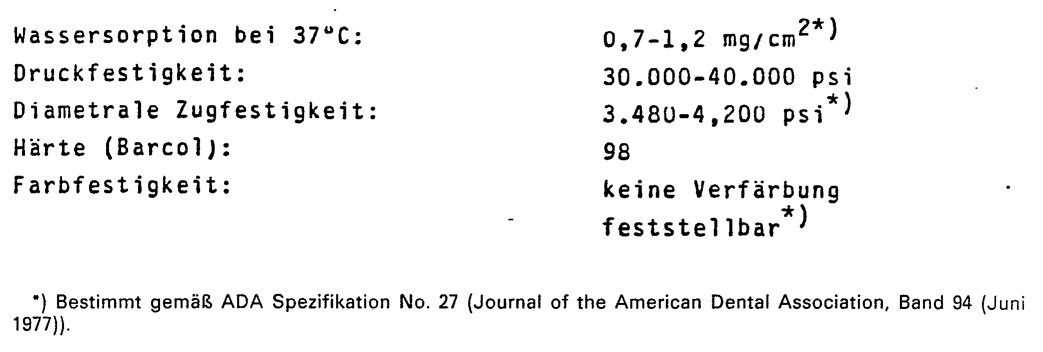

Es wurde nun gefunden, daß diese Eigenschaften der konventionellen, bekannten Composites erheblich verbessert werden konnen durch die Verwendung neuer Monomerer gemäß der Erfindung, was wahrscheinlich durch eine deutliche Erhöhung des Verhältnisses von anorganischem Füllstoffgehalt zum Harzgehalt verursacht wird. Dies hat eine Verbesserung der physikalischen Eigenschaften der ausgehärteten Füllungen, insbesondere im Hinblick auf die Härte und die Abrasionsfestigkeit, zur Folge.

Der Füllstoffgehalt in den Füllungsmaterialien gemäß der Erfindung kann dabei bis zu ca. 90 % erhöht werden.

Es ist deshalb ein Ziel der Erfindung, ein dentales Füllungsmaterial herzustellen, das gekennzeichnet ist durch einen Anteil von mindestens einem der neuen Monomeren gemäß der Erfindung, mindestens einem anorganischen Füllstoff, einem Polymerisationsinitiator oder -beschleuniger sowie gegebenenfalls weiteren Verbindungen, die in diesen Zusammensetzungen bekannt sind, wie andere Monomeren, UV-Absorbern, Stabilisatoren, Pigmenten und Farbstoffen etc..

Die verwendeten anorganischen Füllstoffe können röntgenstrahlendurchlässig oder -undurchlässig sein Geeignete Produkte sind die verschiedenen Silikate wie Glas (pulverisiertes Glas), Quarz, Borosilikatglas und andere Gläser wie Quarzit, Cristobalit, etc.. Geeignete röntgenopake Füllstoffe sind Bariumaluminiumsilikat, Lithiumaluminiumsilikat oder Glaskeramik-Füllstoffe, die z.B. die Elemente Lanthan oder Zirkonium enthalten Geeignete röntgenopake Füllstoffe sind z.B. in den US -Patenten Nr. 3,801,344, 3, 808,170 und 9,975,203 sowie in der DE-A Nr. 2,347,591 beschrieben.

Um die Verträglichkeit des anorganischen Füllmaterials mit den organischen Monomeren zu verbesseren. können die Füllstoffe in an sich bekannter Weise silanisiert werden.

Die Teilchengröße der anorganischen Füllstoffe liegen normalerweise zwischen ca. 0.01 und 100 Mikron. In vielen Fällen ist es möglich und auch sinnvoll, Kombinationen von Füllstoffen mit großen und kleinen Teilchendurchmessern zu verwenden, wobei der bevorzugte Durchmesser zwischen ca. 0,05 und ca. 50, besonders ca. 20 Mikron liegt.

Geeignete Füllstoffe werden auch in der Europäischen Offenlegungsschrift Nr. 60 911 und der US-PS Nr 4,388,069 beschrieben.

Im Prinzip können Composites in zwei Modifikationen verwendet werden, entweder als Zwei-Phasen-Zusammensetzungen oder als Ein-Phasen-Zusammensetzung.

Bei den Zwei-Phasen-Zusammensetzungen enthält eine Phase einen Polymerisationsinitiator, z.B. ein Peroxid, und die andere Phase einen Beschleuniger, z.B. ein organisches Amin.

Beide Phasen werden kurz vor dem Füllen des Zahns zusammengebracht; die Polymerisation (Härtung) wird in der offenen Kavität durchgeführt, die zuvor mit einer Unterfüllung versehen wurde, bzw. mit einem Haftvermittler.

Ein-Phasen-Präparate polymerisieren unter dem Einfluß von Licht, z.B. UV- oder Laser-Licht und enthalten einen Photopolymerisationsinitiator und einen Beschleuniger dafür.

Natürlich ist der Gebrauch der neuen Monomeren gemäß der Erfindung sowohl bei Zwei- als auch bei Ein-Phasen-Präparaten möglich.

Wie erwähnt, polymerisieren die Ein-Phasen-Präparate durch Lichteinfluß. Geeignete Photopolymerisationsinitiatoren sind wohlbekannt; bevorzugte Verbindungen für diesen Zweck sind Carbonylverbindungen, besonders Benzil und Benzilderivate wie 4,4-Oxidibenzil oder andere Dicarbonylverbindungen, z.B. Diacetyl, 2,3-Pentandion oder Metallcarbonyle, Chinone und ihre Derivate, etc.. Der Anteil an Photopolymerisationsinitiatoren in der gesamten Zusammensetzung beträgt ca. 0,01 bis ca. 5 Gewichts-%.

Diese Ein-Phasen-Präparate enthalten vorzugsweise auch sogenannte Polymerisationsbeschleuniger. Dieses sind Substanzen, die die Polymerisationsreaktion beim Vorhandensein von Polymerisationsinitiatoren aktivieren.

Bekannte Beschleuniger sind z.B. verschiedene Amine wie p-Toluidin, Dimethyl p-toluidin, Trialkylamine, Polyamine wie N,N,N',N'-Tetraalkylalkylendiamine und Sulfimide, vorzugsweise in einer Menge von ca. 0,01 bis ca. 5 % der gesamten Zusammensetzung.

Falls das dentale Restorationsmaterial, das die neuen Monomeren gemäß der Erfindung enthält, nicht lichthärtend sein und in zwei Phasen vorliegen soll, die bis zu ihrer Anwendung getrennt sind, enthält eine dieser Mischungen einen Polymerisationsinitiator.

Dies sind vorzugsweise Peroxide, die Radikale bilden, wenn sie die Polymerisation der ungesättigten Verbindungen initiieren. Geeignete Peroxide sind z.B. Acylperoxide wie Benzoylperoxid, Cumolhydroperoxid, Harnstoffperoxid, tert.-Butylhydroperoxid oder Perbenzoate und Silylperoxide, vorzugsweise in einer Menge von 0,01 bis ca. 5, insbesondere ca. 0,5 bis ca. 2,5 Gewichts-% der gesamten Zusammensetzung.

Falls eine Phase des Zwei-Phasen-Materials einen Polymerisationsinitiator enthält, ist es angebracht, der anderen Phase einen Beschleuniger vom oben beschriebenen Typ hinzuzufügen, vorzugsweise ein Amin.

In den dentalen Restorationsmaterialien, die die neuen Monomeren nach der Erfindung enthalten, können polymerisierbare Organosilicone verwendet werden, wie Methacroylalkyltrihydroxysilan oder Methacroyltrimethoxysilan, um die Haftung zwischen anorganischem Füllstoff und Harz zu verbessern.

Zusätzlich zu den neuen Monomeren gemäß der Erfindung können andere schon zu diesem Zweck empfohlene Monomere in den dentalen Restorationsmaterialien verwendet werden. Solche Monomere sind z.B. Alkandioldimethacrylate wie 1,6-Hexandioldimethacrylat, 1,4-Butandioldimethacrylat oder Tri- oder Tetraethylenglycoldimethacrylat Bis-(2-methacroyl-ethyl)phthalat, -isophthalat oder -terephthalat, Trimethylolpropandi- und -trimethacrylat, Reaktionsprodukte aus Diisocyanaten und einfachen Hydroxyalkylmethacrylaten wie z.B. in der DE-A Nr. 2,312,559 beschrieben, Reaktionsprodukte von Bisphenolen, besonders Bisphenol A, und Glycidylmethacrylat (Bis-GMA), Addukte von (Di)Isocyanaten und 2,2-Propan-bis-3- (4-phenoxy)-1,2-hydroxypropan-1-methacrylat gemäß der US-PS Nr. 3,629,187 und andere geeignete polymerisierbare Verbindungen.

Geeignete Monomere sind auch Addukte von Methacroylalkyläthern, -alkoxybenzolen und/oder -alkoxycycloalkanen und Diisocyanaten entsprechend der EP-OS Nr. 44,352.

Compositematerialien können geringe Mengen von geeigneten Farbstoffen enthalten, um die Farbe der Füllungen der natürlichen Farbe so gut wie möglich anzupassen.

Es kann auch nützlich sein, geringe Mengen von UV-Stabilisatoren zuzusetzen, z.B. Hydrochinon, p-Benzochinon, tert.-Butylhydroxytoluol etc..

Die folgenden Beispiele dienen der Erläuterung der Erfindung:

### Beispiel A

148 g Phthalsäureanhydrid 0 1 g 2,5-Di-tert.-butyl-4-methyl-phenol und 59 g 1,6 Hexandiol werden 2 Std. bei 140- C unter Rühren an einem Rückflußkühler erhitzt. Dann gibt man innerhalb 1 Std. 160 g Glycidylmethacrylat zu, wobei man die Temperatur auf 130°C hält. Danach wird die Temperatur auf 130°C gehalten, bis die Säurezahl der Mischung 0,5 ist. Anschließend wird das nicht reagierte Glycidylmethacrylat unter 110 mm Hg Druck im Vakuum entfernt. Das vollständige Entfernen wird kontrolliert durch Bestimmung des Epoxyäquivalentwertes. Das so erhaltene Reaktionsprodukt ist eine farblose, viskose Flüssigkeit mit der Struktur:

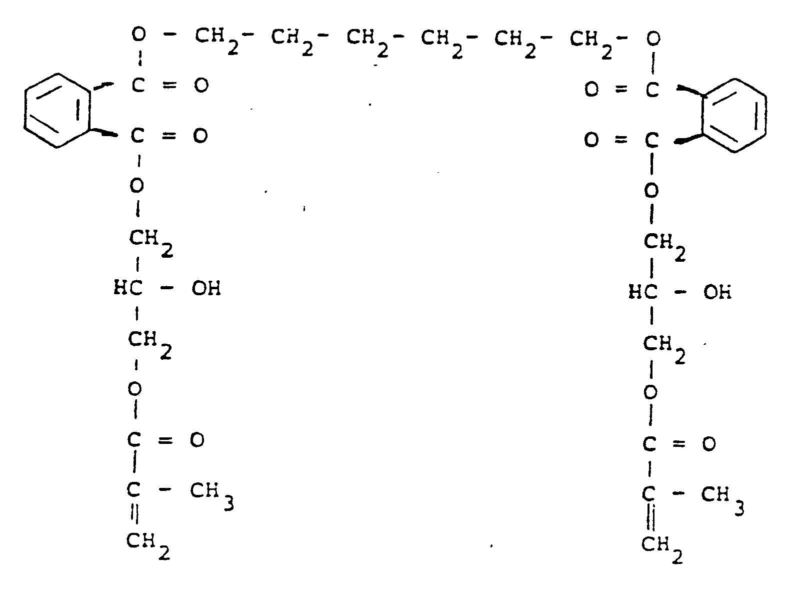

Brechungsindex (30°): 1,525.

Entsprechende Acrylverbindungen werden analog hergestellt.

b) Diese Verbindung kann mit n-Butylisocyanat in Gegenwart von Dibutylzinndiacetat bei 70-80°C 3 Std. lang zum entsprechenden Di-n-butylcarbamat der Formel umgesetzt werden: 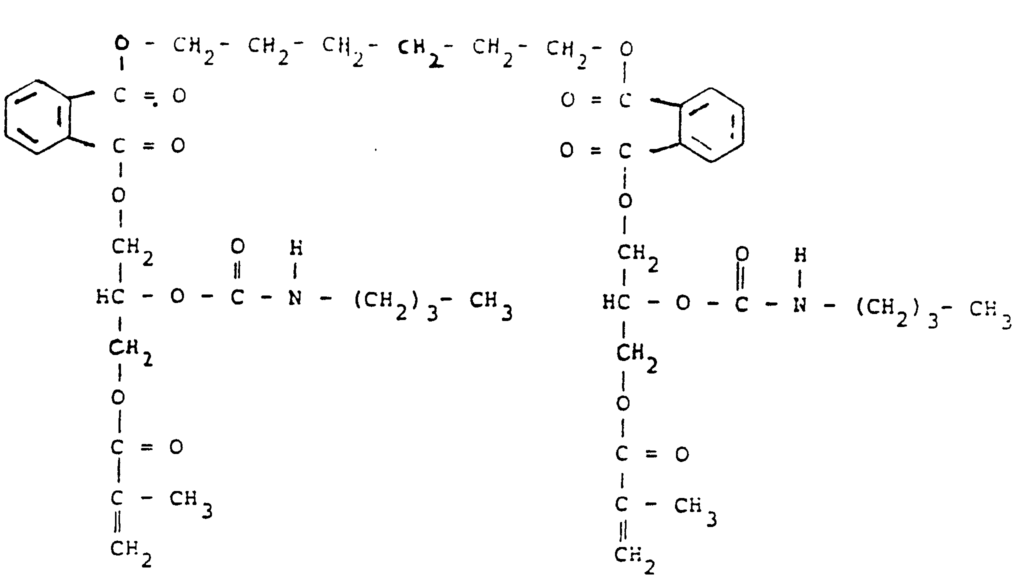
Brechungsindex (30°C): 1,512

In ähnlicher Weise werden die entsprechenden Acrylverbindungen hergestellt.

### Beispiel B

a) Gemäß dem in Beispiel A.a) beschriebenen Verfahren wurde 1,3-Butylenbis -[2-(3'-methacroyl-2'-hydroxypropyl)phthalat] hergestellt aus 1,3-Butandiol, Phthalsäureanhydrid und Glycidylmethacrylat: 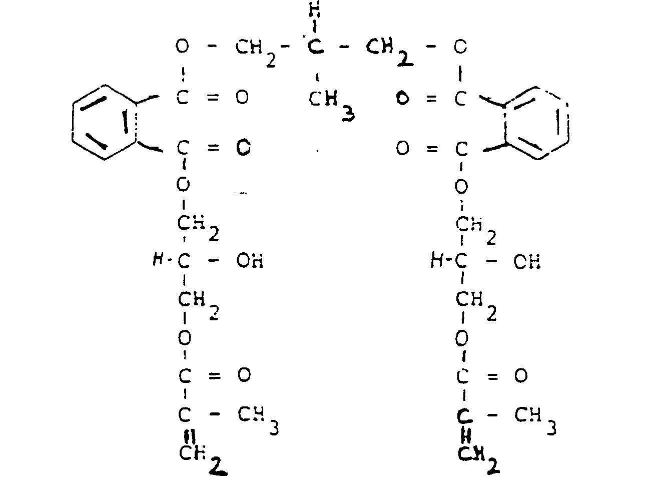 Brechungsindex (25°C): 1,525
b) Zu 226 g der gemäß a) hergestellten Verbindung werden 0,15 g Dibutylzinndiacetat und 79,3 g Phenylisocyanat unter Rühren 1 Std. lang bei 70-80° C hinzugefügt.

Das entsprechende Diphenylcarbamat wird in quantitativer Ausbeute erhalten: 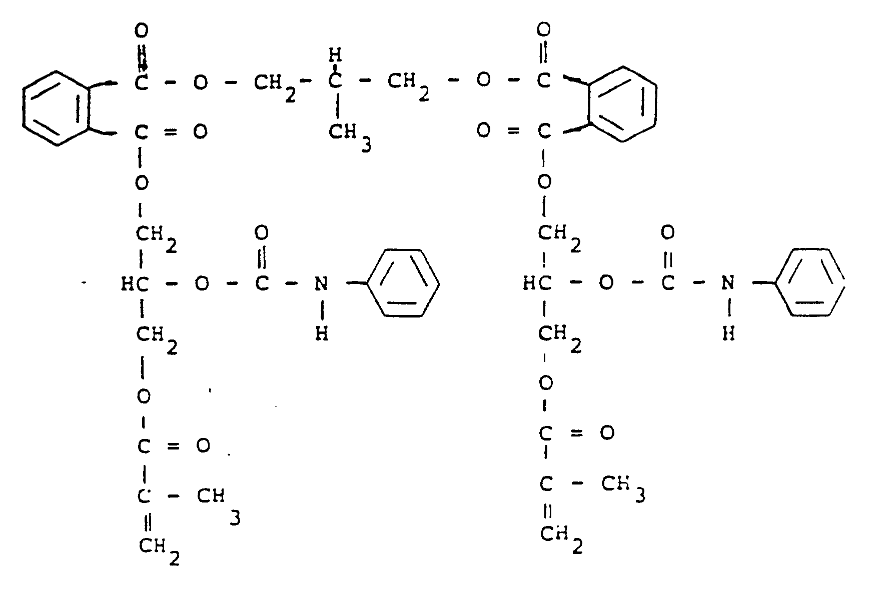
Brechnungsindex (70°C): 1,535

Ebenso wurden die entsprechenden Acrylverbindungen hergestellt.

### Beispiel C

a) 1,4-Cyclohexanbis- (3'-methacroyl-2'-hydroxypropylmaleat) wird gemäß dem in Beispiel A.a) beschriebenen Verfahren aus Maleinsäureanhydrid, 1,4-Cyclohexandiol und Glycidylmethacrylat hergestellt: 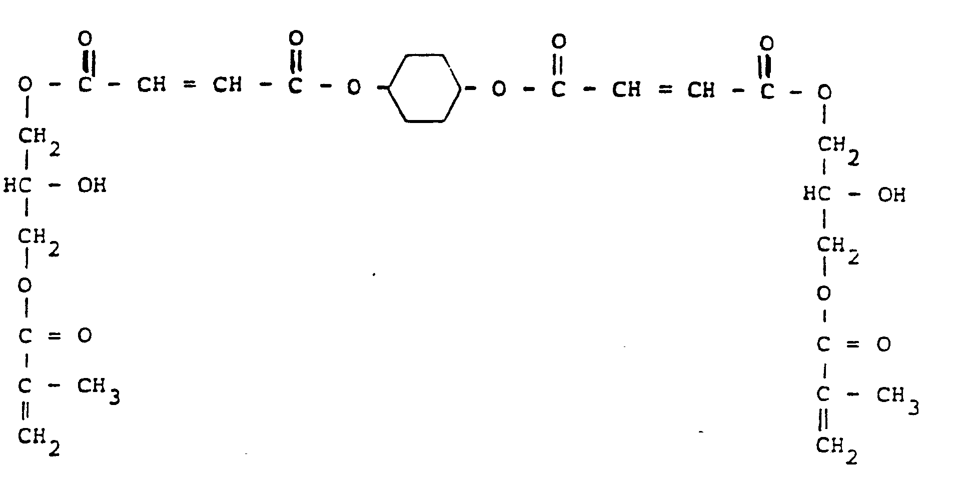 Brechnungsindex (50° C): 1,495
b) Die gemäß a) hergestellte Verbindung wird mit Phenylisocyanat gemäß der in Beispiel B.b) beschriebenen Methode zum entsprechenden Diphenylcarbamat umgesetzt: 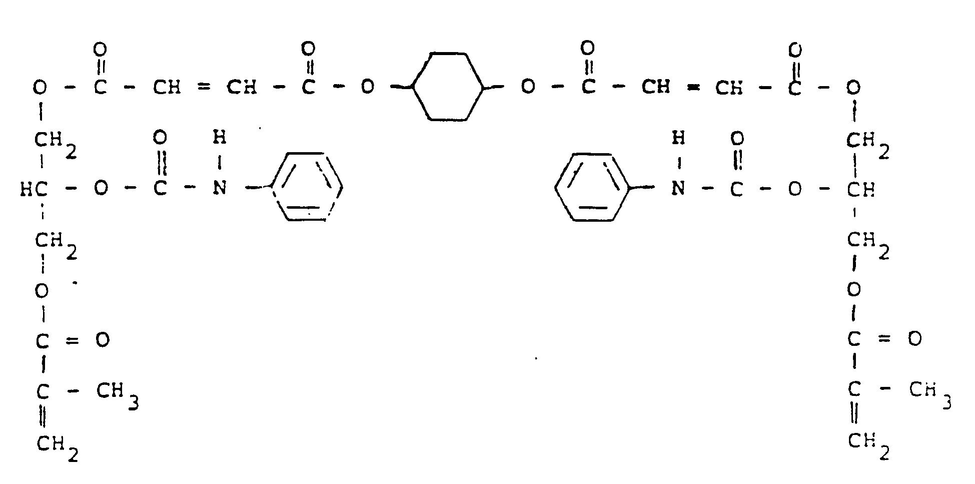
Das Produkt ist bei Zimmertemperatur fest.

### Beispiel D

a) Auf gleiche Weise wie in Beispiel A.a) beschrieben wird 1,4-Butylenbis- 2'-(3"- methacroyl-2"- hydroxypropyl) phthalat aus 1,4-Butandiol, Phthalsäureanhydrid und Glycidylmethacrylat hergestellt: 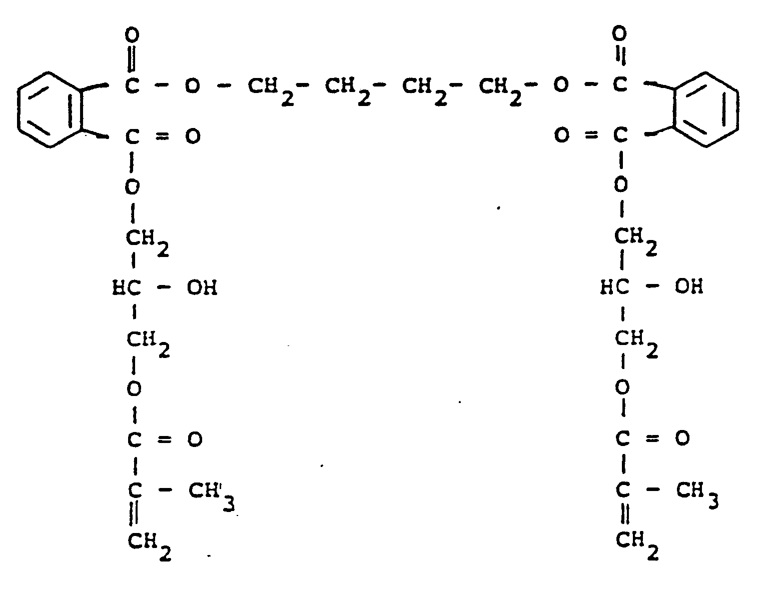
   Brechungsindex (30° C): 1,522
   Analog wurden die entsprechenden Acrylverbindungen hergestellt.
b) Die Verbindung kann in die entsprechenden n-Butyl-, n-Phenyl-, n-Hexyl oder Cyclohexylmono- und dicarbamate überführt werden, beispielsweise durch Reaktion mit n-Butyl-isocyanat wie in Beispiel B.b) beschrieben: 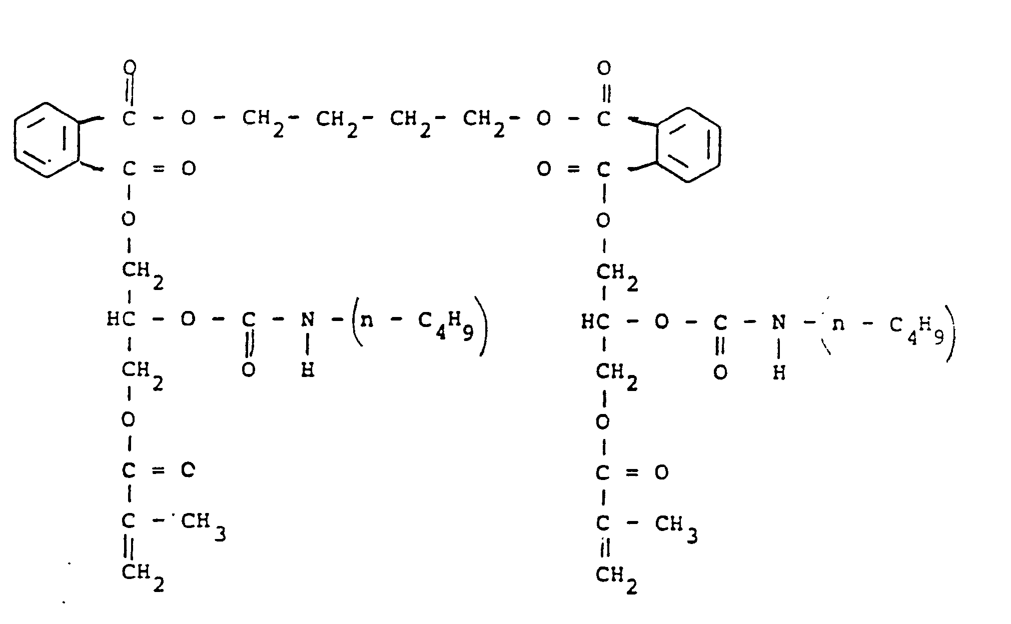 Brechungsindex (30°C): 1,522
   In analoger Weise wurden die entsprechenden Acrylverbindungen hergestellt.

### Beispiel E

a) Gemäß dem in Beispiel A.a) beschriebenen Verfahren wurde aus Norbornen-2,3-dicarbonsäureanhydrid, 1,2-Propylenglycol und Glycidylmethacrylat das 1,2-Propylenbis- ((3'-metha-croyl-2'-hydroxypropyl)-5-norbornen-2,3-dicarboxylat-]hergestellt:

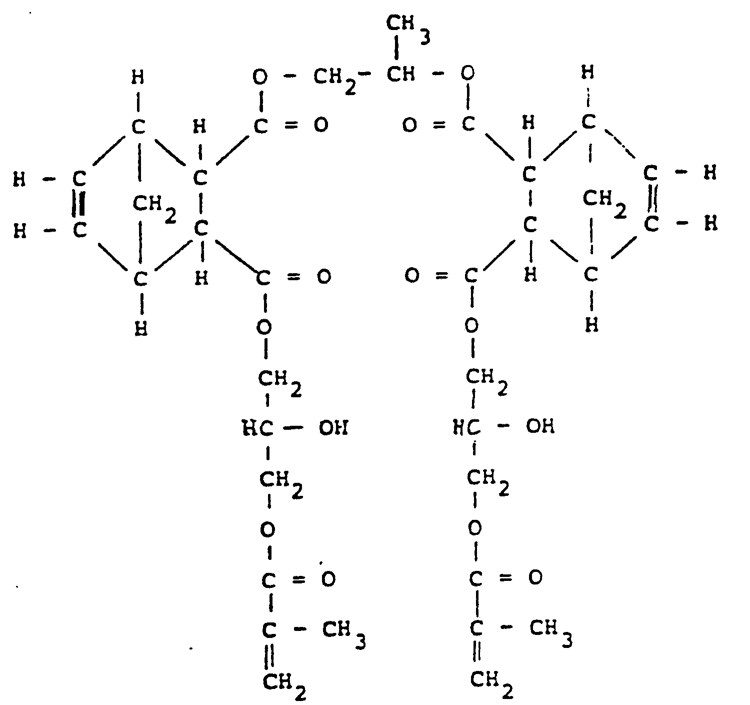

Erläuternde Beispiele für den Einsatz der neuen Monomeren in Dentalprodukten:

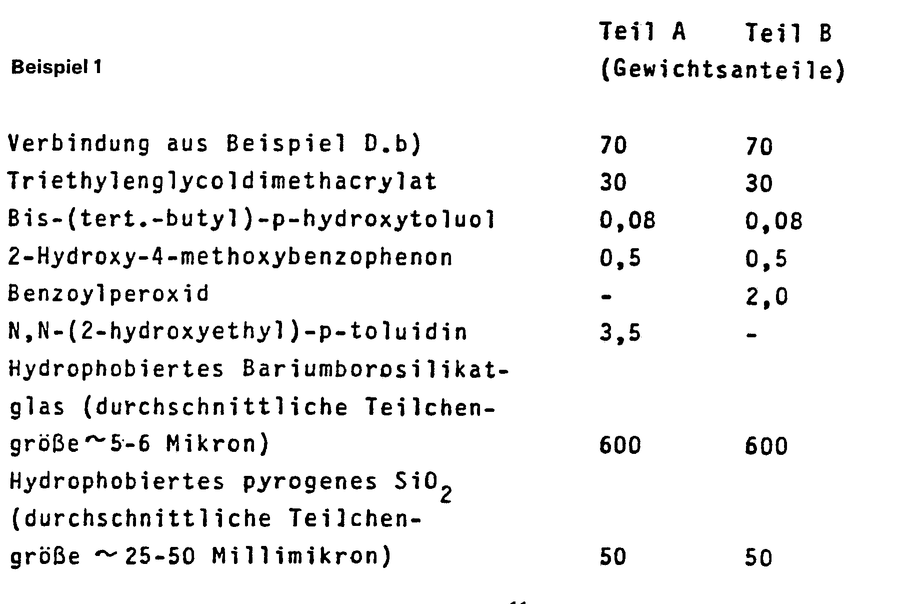

Teil A und B werden zusammengemischt und innerhalb 120 sec. bei 23°C gehärtet. Das gehärtete Material besitzt Lichtdurchlässigkeit und andere optische Eigenschaften, die jenen der Zahnstruktur des Menschen ähneln. Seine sonstigen Eigenschaften waren wie folgt:

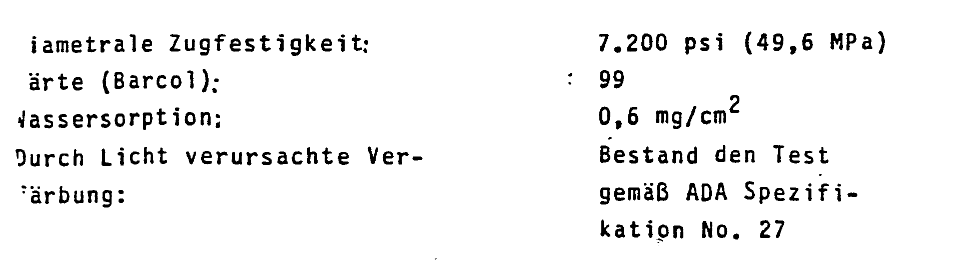

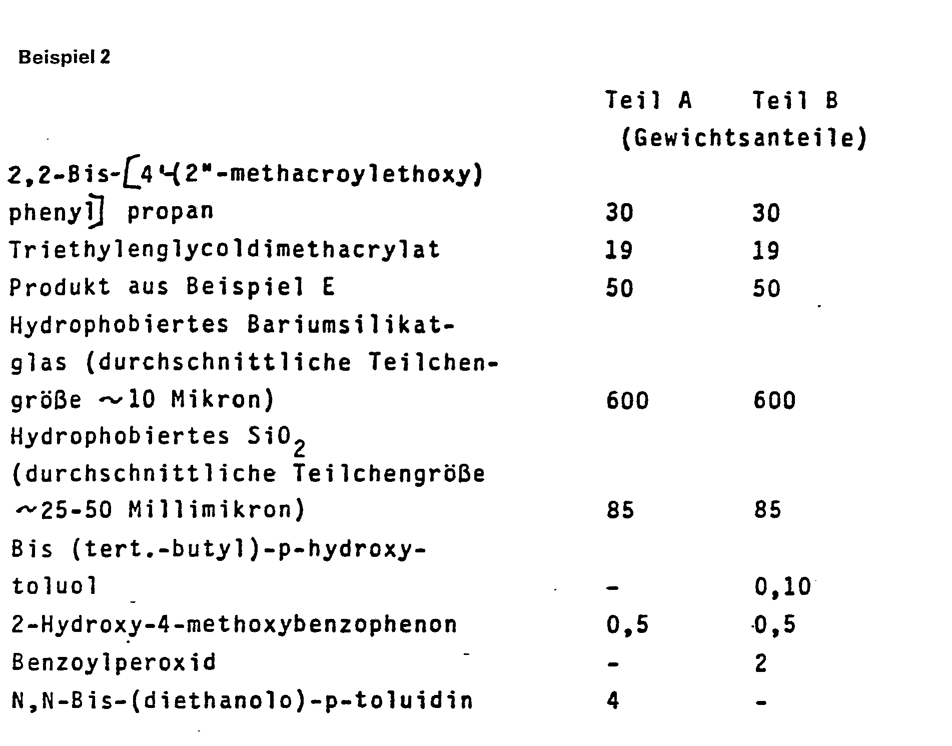

Zur Aushärtung wurden gleiche Mengen von A und B gemischt und 150 sec. bei 25° C polymerisiert.

Das entstandene Polymerisat zeigte folgende Eigenschaften:

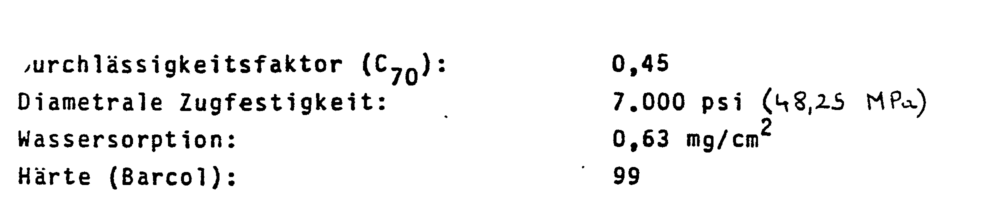

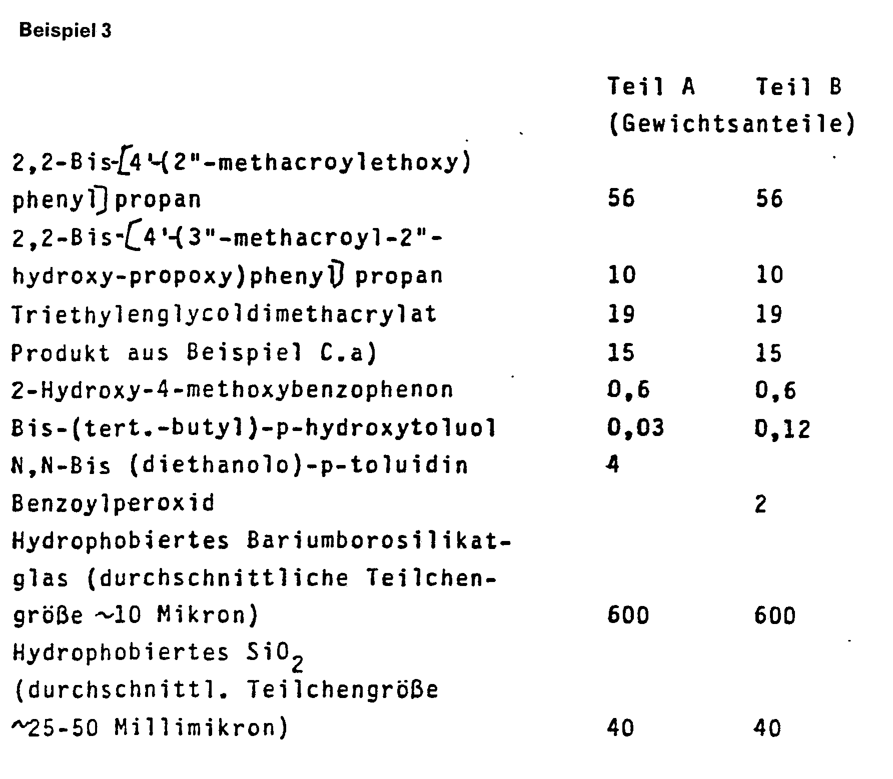

Zur Aushärtung wurden gleiche Mengen von A und B gemischt und 250 sec. bei 23° C polymerisiert. Das entstandene Polymerisat zeigte folgende Eigenschaften:

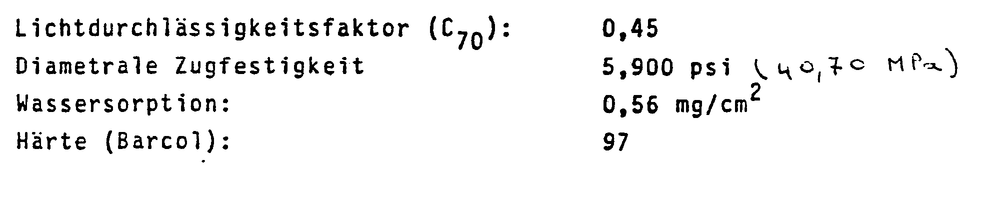

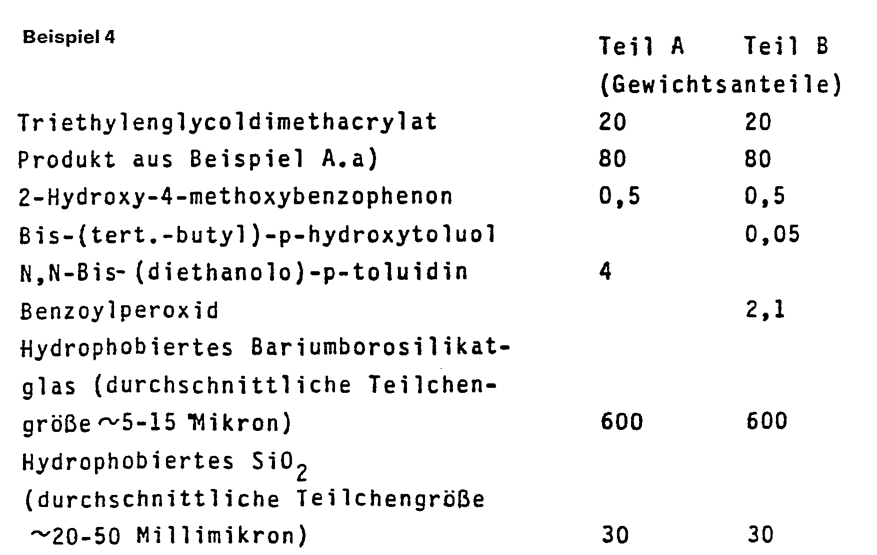

Zur Aushärtung wurden gleiche Mengen von A und B gemischt und 150 sec. bei 23°C polymerisiert. Das entstandene Polymerisat hat folgende Eigenschaften:

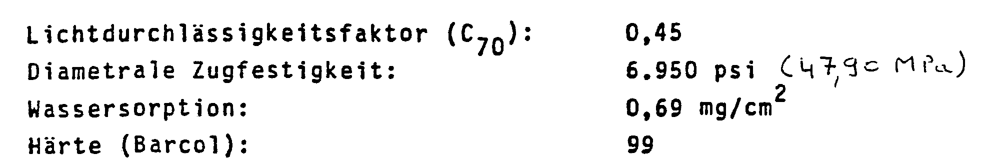

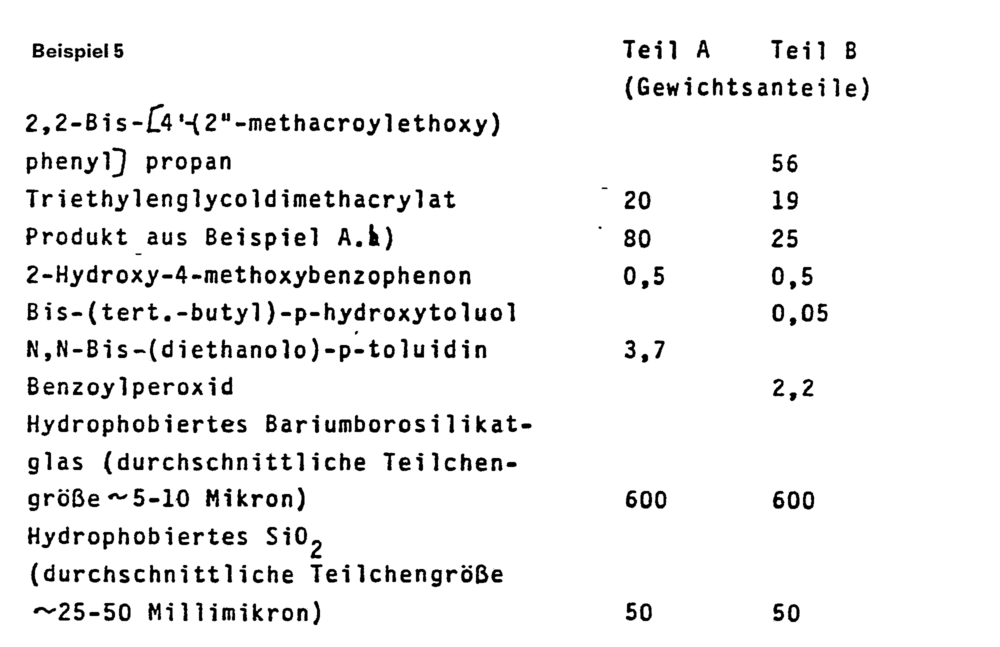

Zur Aushärtung wurden gleiche Mengen von A und B gemischt und 150 sec. bei 23° C polymerisiert. Das entstandene Polymerisat zeigte folgende Eigenschaften:

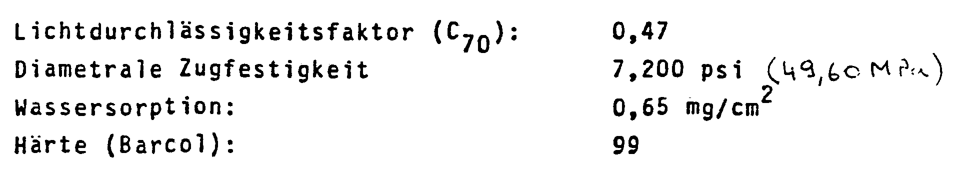

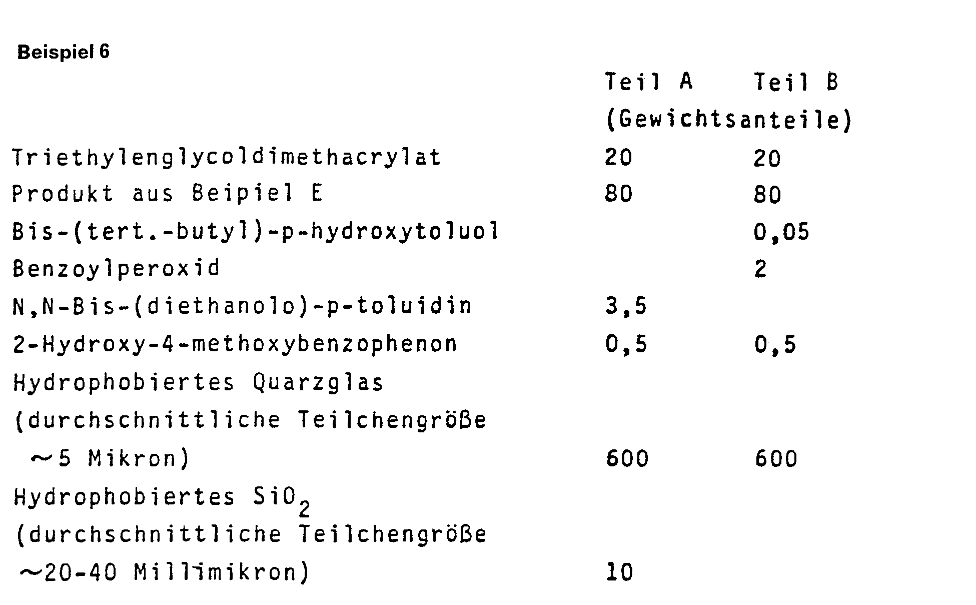

Zur Aushärtung wurden gleiche Mengen von A und B gemischt und 150 sec. bei 23°C polymerisiert. Das entstandene Polymerisat zeigte folgende Eigenschaften:

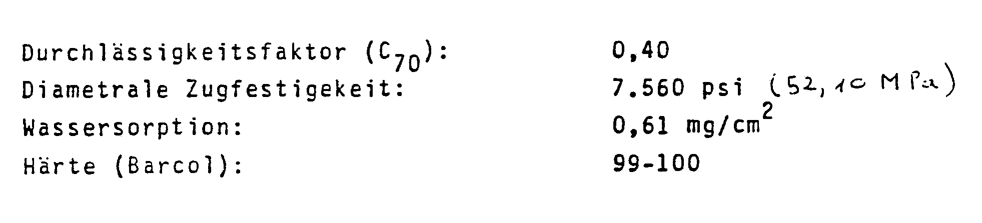

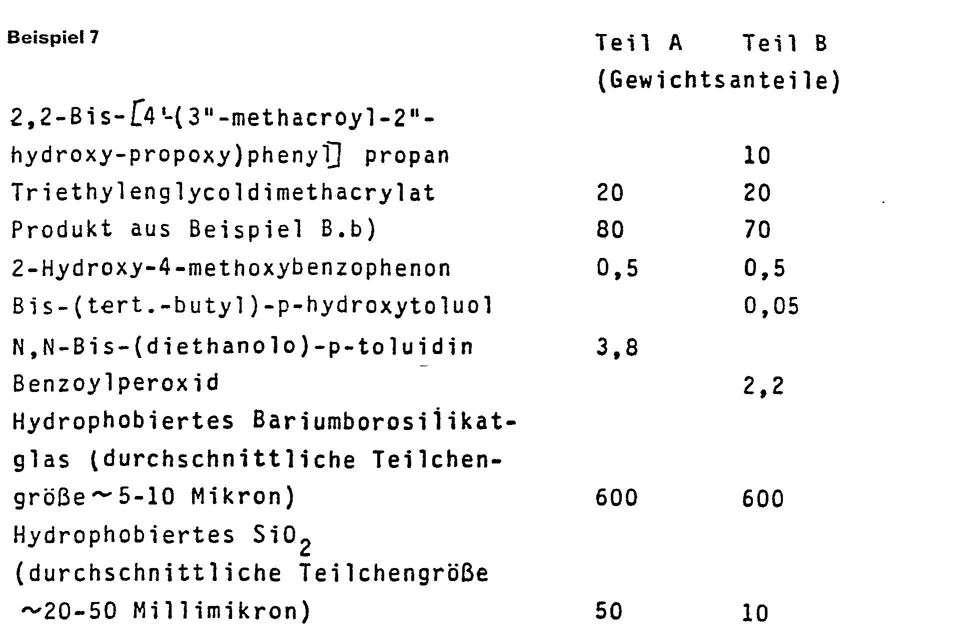

Zur Aushärtung wurden gleiche Mengen von A und B gemischt und 150 sec. bei 23°C polymerisiert. Das enstandene Polymerisat zeigte folgende Eigenschaften:

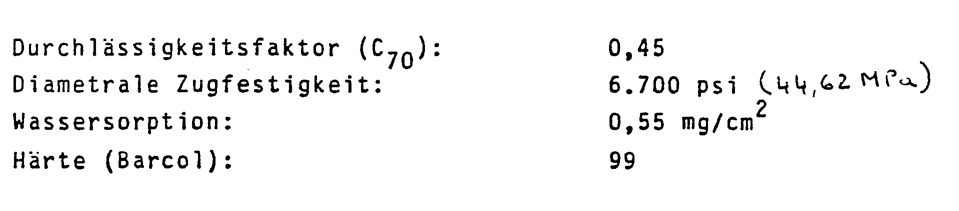

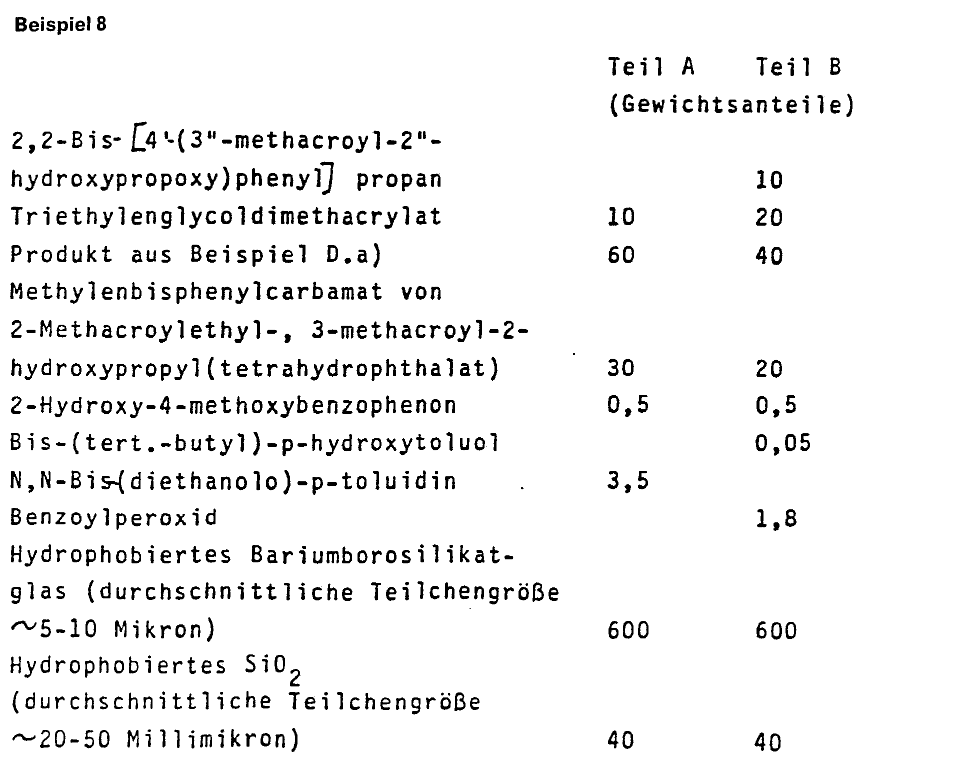

Zur Aushärtung wurden gleiche Mengen von A und B gemischt und 150 sec. bei 23°C polymerisiert. Das entstandene Polymerisat zeigte folgende Eigenschaften:

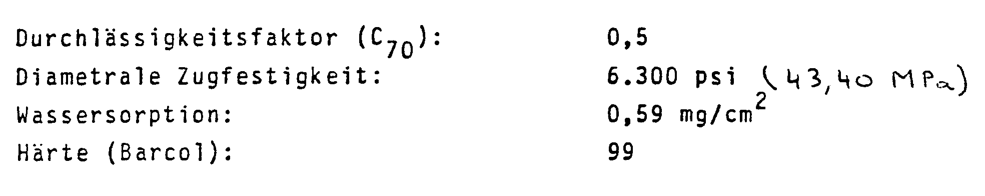

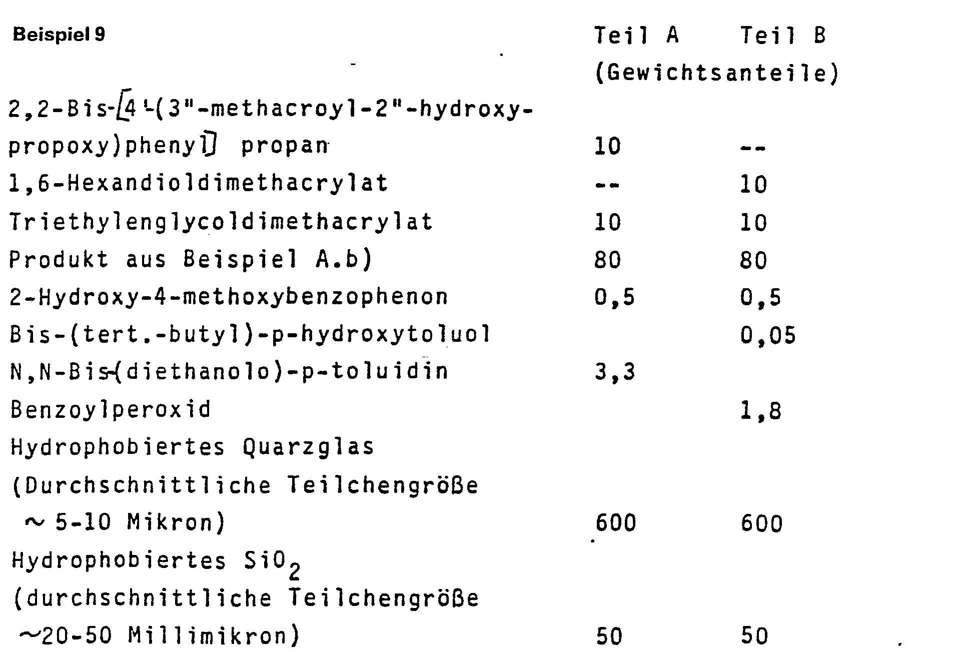

Zur Aushärtung wurden gleiche Mengen von A und B gemischt und 150 sec. bei 23" C polymerisiert. Das entstandene Polymerisat zeigte folgende Eigenschaften:

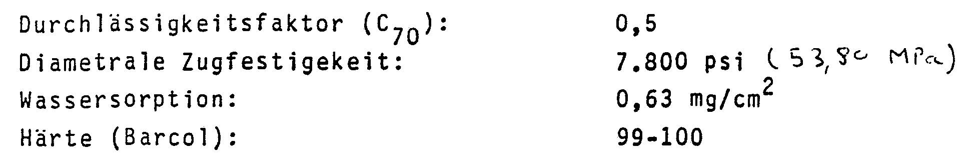

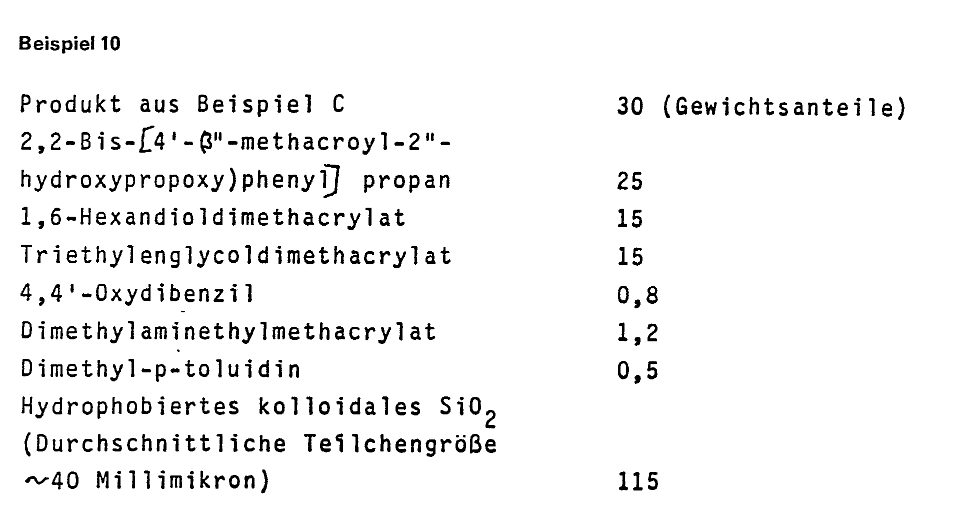

Die Mischung ist lichthärtend mit guter Härtungstiefe und zeigt nach dem Härten ausgezeichnete mechanische Werte und hohe Polierbarkeit.

### Beispiel 11

Im folgenden wird ein Beispiel für einen Dentallack gegeben, der mit UV-Licht härtet:

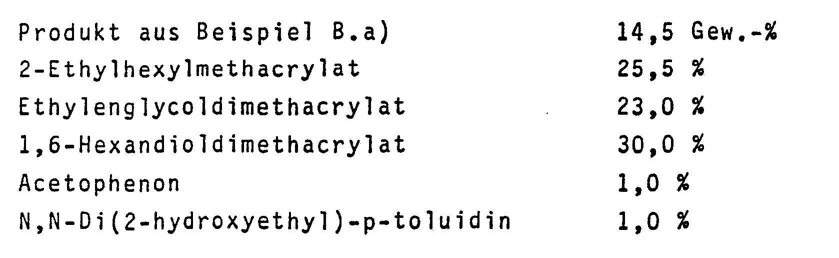

## Patentansprüche

1. Diacryl- und Dimethacrylsäureester der allgemeinen Formel 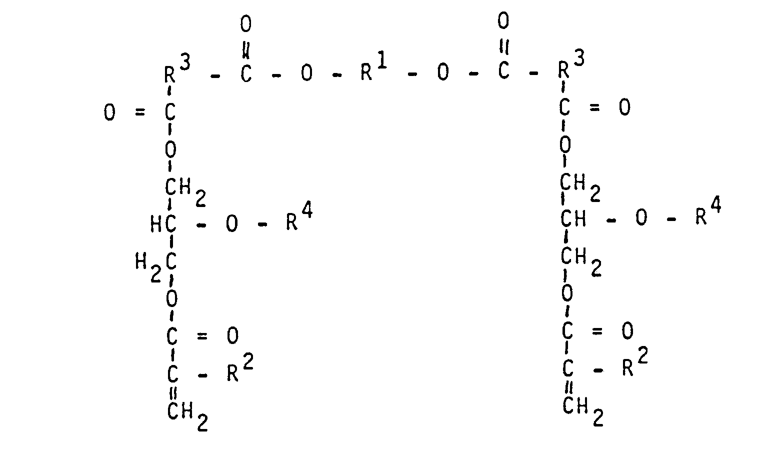 wobei R¹ eine Ethylen-, Propylen-, Butylen-, Hexamethylen-, Phenylen-, Toluylen-, Methylenbisphenyl-, Propylenbisphenyl-Cyclohexan-, Dicyclopentandimethylen-, Methylenbiscyclohexyl- oder Propylenbiscyclohexylgruppe; R² Wasserstoff oder Methyl; R³ einen -CH=CH-, -CH₂CH₂-, -CH₂CH₂CH₂-, -CH₂CH₂CH₂CH₂-Rest -Rest, einen gegebenenfalls substituierten oder hydrierten Benzolring, einen Cyclohexan-, Cyclohexen- oder einen Cis-Norbornen-Rest, und R⁴ Wasserstoff oder die Gruppe 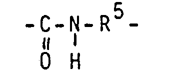 bedeuten, wobei R⁵ n-Butyl, Isobutyl, Hexyl oder einen gegebenenfalls substituierten Phenyl- oder Cyclohexylrest darstellt.

2. Verbindungen der Formel nach Anspruch 1, wobei R einen -CH = CH-Rest darstellt.

3. Verbindungen der Formel nach Anspruch 1, wobei R³ einen Benzolrest darstellt.

4. Verbindungen der Formel nach Anspruch 1, wobei R³ einen Tetrahydrobenzolrest darstellt.

5. Verbindungen der Formel nach Anspruch 1, wobei R³ einen Cyclohexanrest darstellt.

6. Verbindungen der Formel nach einem der Ansprüche 1 bis 5, wobei R⁴ Wasserstoff darstellt.

7. Verbindungen der Formel nach einem der Ansprüche 1 bis 5, wobei R⁴ einen 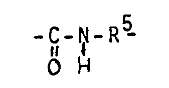 Rest darstellt und R⁵ einen n-Butyl-, Isobutyl-, Hexyl-, gegebenenfalls substituierten Phenyl- oder Cyclohexylrest darstellt.

8. Verwendung einer oder mehrerer Verbindungen gemäß den Ansprüchen 1 bis 7 als Bindemittel in Dentalmaterialien.

9. Dentales Restorations- und Füllungsmaterial, das mindestens einen anorganischen Füllstoff, mindestens einen Polymerisationsinitiator und/oder mindestens einen Polymerisationsbeschleuniger und wahlweise andere Bestandteile, die in solchen Zusammensetzungen gebräuchlich sind, enthält, gekennzeichnet durch den Gehalt an mindestens einer Verbindung nach den Ansprüchen 1 bis 7.

10. Dentales Restorations- und Füllungsmaterial nach Anspruch 9, gekennzeichnet durch einen Gehalt an folgenden Bestandteilen:
5 bis 50 Gewichts-% mindestens einer der Verbindungen nach den Ansprüchen 1 bis 7;
0,1 bis 5,0 Gewichts-% mindestens eines Polymerisationsinitiators und/oder Polymerisiationsbeschleunigers; 50 bis 90 Gewichts-% mindestens eines Füllstoffs; sowie gegebenenfalls zusätzlichen polymerisierbaren Verbindungen, UV-Absorbern, Farbstoffen, Haftungsverbesserern und sonstigen in diesen Präparaten üblichen Bestandteilen.

## Claims

1. Diacrylic and dimethylacrylic acid esters of the general formula 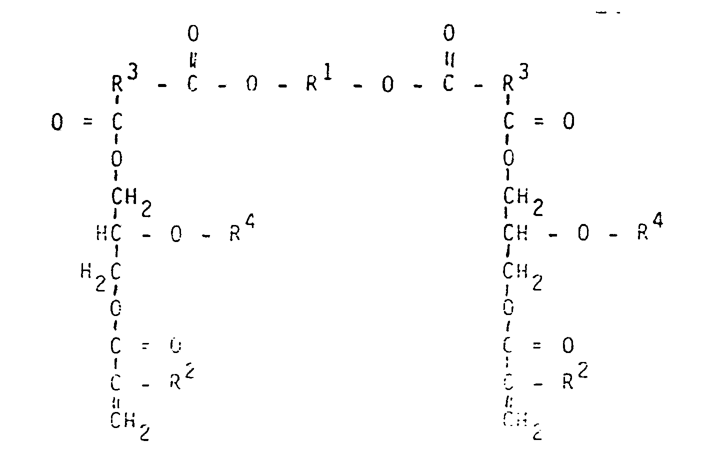 where R¹ means an ethylene, propylene, butylene, hexamethylene, phenylene, toluylene, methylene bisphenyl, propylene bisphenyl, cyclohexane, dicyclopentane dimethylene, methylene biscyclohexyl or propylene biscyclohexyl group; R² means hydrogen or methyl; R³ means a -CH=CH-,-CH₂CH₂- or -CH₂CH₂CH₂CH₂ -group; an optionally substituted or hydrogenated benzene ring, a cyclohexane, cyclohexene or a cis-norbornene group, and R⁴ means hydrogen or the group 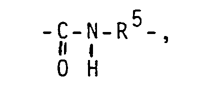 where R⁵ represents n-butyl, isobutyl or a possibly substituted phenyl or cyclohexyl group.

2. Compounds of the formula according to claim 1, where R³ represents a -CH = CH-group.

3. Compounds of the formula according to claim 1, where R³ represents a benzene group.

4. Compounds of the formula according to claim 1, where R³ represents a tetrahydrobenzene group.

5. Compounds of the formula according to claim 1, where R³ represents a cyclohexane group.

6. Compounds of the formula according to one of claims 1 to 5, where R⁴ represents hydrogen.

7. Compounds of the formula according to one of claims 1 to 5, where R⁴ represents a 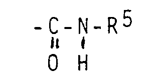 group and R⁵ represents a n-butyl, isobutyl, hexyl, an optionally substituted phenyl or cyclohexyl group.

8. Use of one or more compounds according to claims 1 to 7 as bonding agent in dental materials.

9. Dental restoration and filling material containing at least one inorganic filler, at least one polymerization initiator and/or at least one polymerization accelerator and optionally other components, used in such compositions, characterized by the presence of at least one compound according to claims 1 to 7.

10. Dental restoration and filling material according to claim 9, comprising the following components:
5 to 50 % by weight of at least one of the compounds according to claims 1 to 7;
0,1 to 5,0 % by weight of at least one polymerization initiator and/or polymerization accelerator;
50 to 90 % by weight of at least one filler; as well as possibly additional polymerizable compounds, UV- absorbers, dyestuffs, adhesion-improving agents and other components normally used in these preparations.

## Revendications

1. Diesters d'acide acrylique et methacrylique de la formule générale 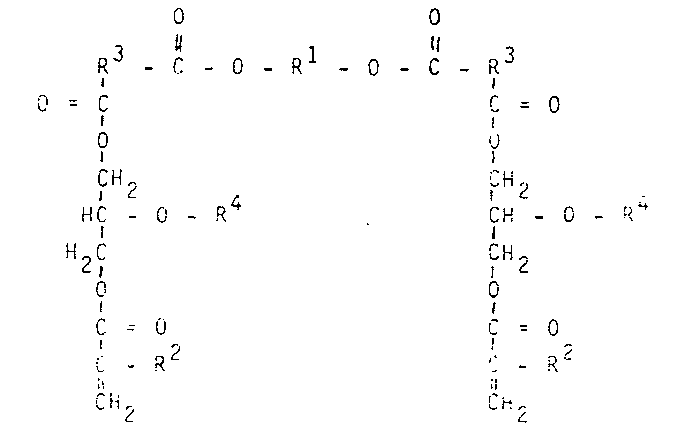 où R¹ représente un groupe d'éthylène, de pyopylène, de butylène, d'hexaméthylène, de pnénylène, de toluylène, de méthylène bisphényl, de propylène bispnényl, de cyclohexane, de di-cyclopentane bisméthylène, de méthylène biscyclohexyl ou de propylène biscyclohexyl; R² représente l'hydrogène ou méthyl; R³ représente un résidu de -CH =CH-, -CH₂CH₂-, -CH₂CH₂CH₂-, -CH₂CH₂CH₂CH₂-, un groupe de benzène, le cas échéant, substitué ou hydraté, un résidu de cyclohexane, de cyclohexène ou de cis-norbonène, et R⁴ représente l'hydrogène ou le groupe 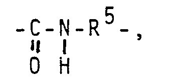 où R⁵ représente n-butyl, isobutyl, hexyl ou un résidu de phényl ou cyclonexyl, le cas échéant, substitué.

2. Composés de la formule selon revendication 1, d'où R³ représente un résidu de -CH=CH-.

3. Composés de la formule selon revendication 1, d'où R³ représente un résidu de benzène.

4. Composés de la formule selon revendication 1, d'où R³ représente un résidu de tetrahydrobenzéne.

5. Composés de la formule selon revendication 1, d'où R³ représente un résidu de cyclohexane.

6. Composés de la formule selon une des revendications 1 à 5, d'où R⁴ représente l'hydrogène.

7. Composés de la formule selon une des revendications 1 à 5, où R⁴ représente un résidu de 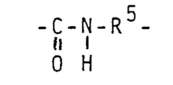 et R⁵ représente un résidu de n-butyl, d'isobutyl, d'hexyl, de phenyl ou cyclonexyl, le cas échéant, substitué.

8. L'emploi d'une ou de plusieurs combinaisons selon les revendications 1 à 7 comme liant dans des matériaux dentaires.

9. Matériau de restauration et d'obturation dentaire qui contient au minimum une matière d'obturation inorganique, au minimum un initiateur de polymérisation et/ou au minimum un accélérateur de polymérisation et facultativement d'autres constituants usuels dans ces compositions, charactérisé par le fait qu il contient au minimum une des composés selon les revendications 1 à 7.

10. Matériau de restauration et d'obturation dentaire selon revendication 9, charactérisé par le fait qu'il contient les constituants suivants:
5 à 50 % de poids d'une ou plusieurs des composés selon les revendications 1 à 7;
0,1 à 5,0 % de poids d'un initiateur de polymérisation et/ou d'un accélérateur de polymérisation; 50 à 900/0 de poids d'un matériau d'obturation; ainsi que, le cas échéant, d'additionnelles composés, d'absorbeurs d'UV, des colorants, des matières à improuver l'adhésion et d'autres constituants usuels dans ces préparations.
